# EUROPEAN PATENT APPLICATION

(11) **EP 1 095 655 A2**
(43) Date of publication of application: **02.05.2001**
(21) Application number: 00309363.0
(22) Date of filing: 24.10.2000
(51) Int. Cl.: A61K 31/54, A61K 31/535, A61K 31/445, A61K 31/435, A61K 31/40

(54) **NK-1 receptor antagonists and eletriptan for the treatment of migraine**

(30) Priority: 25.10.1999 US 161284 P; 10.11.1999 US 164896 P
(71) Applicant: Pfizer Products Inc., Groton, Connecticut 06340 (US)
(72) Inventor: Sobolov-Jaynes, Susan Beth, Groton, Connecticut 06340 (US)
(74) Representative: Ruddock, Keith Stephen

(57) **Abstract**

The present invention relates to a method of treating or preventing migraine in a mammal, including a human, by administering to the mammal eletriptan or a pharmaceutically acceptable salt of eletriptan and an NK-1 receptor antagonist (*e*.*g*., a substance P receptor antagonist). It also relates to pharmaceutical compositions containing a pharmaceutically acceptable carrier, eletriptan or a pharmaceutically acceptable salt of eletriptan and an NK-1 receptor antagonist.

## Description

### Background Of The Invention

The present invention relates to a method of treating migraine in a mammal, including a human, by administering to the mammal eletriptan and a CNS-penetrant NK-1 receptor antagonist (e.g., a substance P receptor antagonist). It also relates to pharmaceutical compositions containing a pharmaceutically acceptable carrier, eletriptan and a CNS-penetrant NK-1 receptor antagonist.

### Summary Of The Invention

The present invention relates to a pharmaceutical composition for the treatment of migraine comprising: (a) eletriptan, 3-(1-methyllpyrrolidin-2(R)-yl]methyl)-5-(2-phenylsulphonylethyl)-1H-indole, or a pharmaceutically acceptable salt of eletriptan; (b) a CNS-penetrant NK-1 receptor antagonist or pharmaceutically acceptable salt thereof; and (c) a pharmaceutically acceptable carrier, wherein the active agents "a" and "b" above are present in amounts that render the combination of the two agents effective in treating migraine.

Eletriptan is a 5HT_{1B/1D} receptor agonist and has been shown to be highly effective for the treatment of migraine. Eletriptan has also been disclosed for the treatment of hypertension, emesis, depression, anxiety, an eating disorder, obesity, drug abuse, cluster headache, pain chronic paroxysmal hemicrania and a headache associated with a vascular disorder.

This invention also relates to a method of treating migraine in a mammal, comprising administering to said mammal an antimigraine effective amount of a pharmaceutical composition comprising: (a) eletriptan or a pharmaceutically acceptable salt of eletriptan; (b) a CNS-penetrant NK-1 receptor antagonist or pharmaceutically acceptable salt thereof and (c) a pharmaceutically acceptable carrier, wherein the active agents "a" and "b" above are present in amounts that render the combination of the two agents effective in treating migraine.

This invention also relates to a method of treating migraine in a mammal, comprising administering to said mammal: (a) eletriptan or a pharmaceuctially acceptable salt of eletriptan: and (b) a CNS-penetrant NK-1 receptor antagonist or a pharmaceutically acceptable salt thereof, wherein the amounts of the two active agents (a) and (b)that are administered are selected so as to render the combination of such two active agents effective in the treatment of migraine.

Examples of NK-1 receptor antagonists that can be used in the methods and pharmaceutical compositions of this invention are compounds of the formula 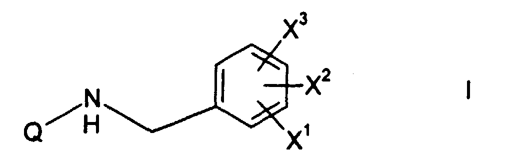 and their pharmaceutically acceptable salts, wherein X¹ is hydrogen, (C₁-C₁₀) alkoxy optionally substituted with from one to three flourine atoms or (C₁-C₁₀) alkyl optionally substituted with from one to three fluorine atoms;
X² and X³ are independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆) alkyl-C(=O)-NH-(C₁-C₆) alkyl, hydroxy(C₁-C₄)alkyl, (C₁-C₄)alkoxy(C₁-C₄)alkyl, -NHC(=O)H and -NHC(=O)-(C₁-C₆) alkyl; and
Q is a group of the formula 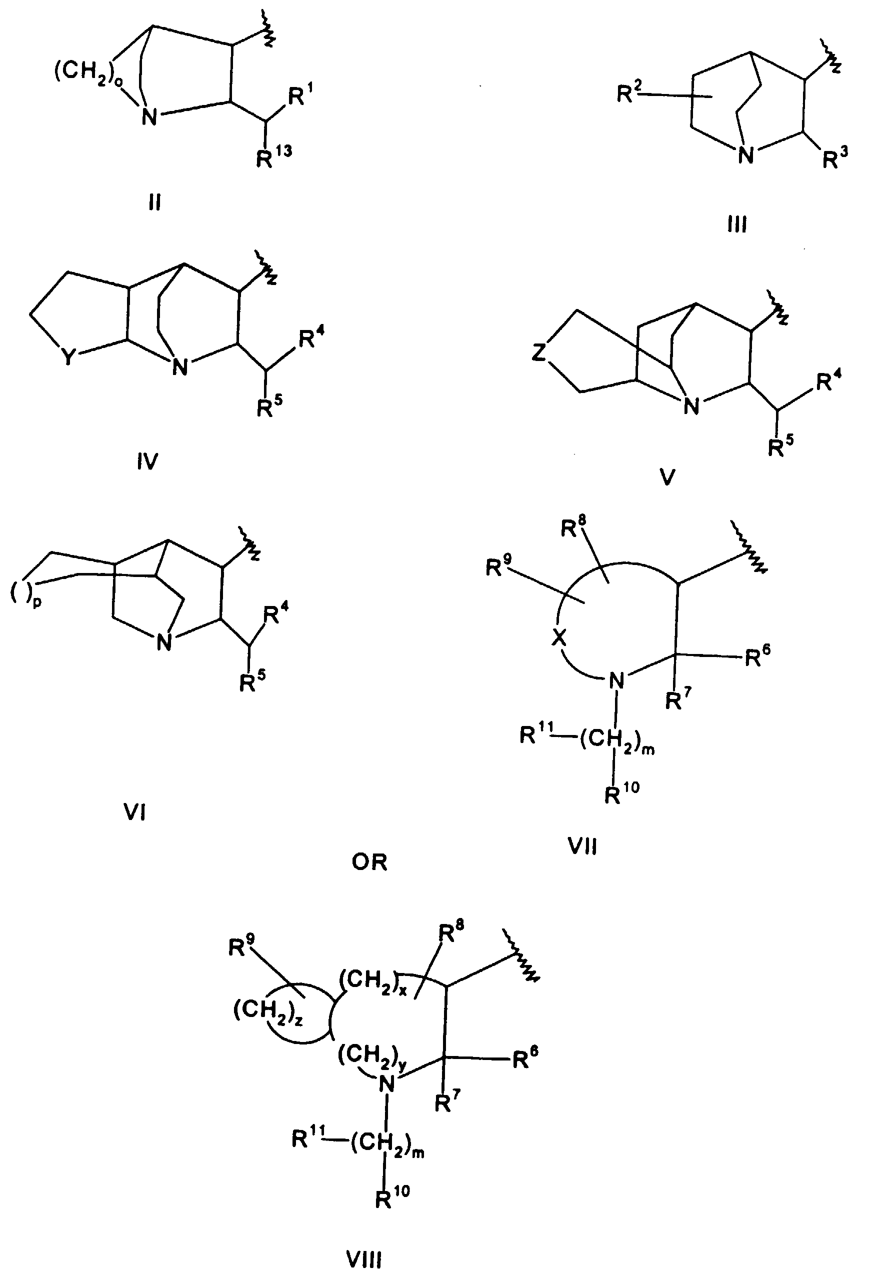 wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R¹³ is selected from (C₃-C₄) branched alkyl, (C₅-C₆) branched alkenyl, (C₅-C₇) cycloalkyl, and the radicals named in the definition of R¹;
R² is hydrogen or (C₁-C₆) alkyl;
R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 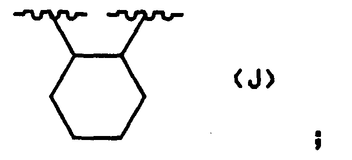
Z is oxygen, sulfur, amino, (C₁-C₃)alkylamino or (CH₂)ₙ wherein n is zero, one or two;
o is two or three;
p is zero or one;
R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, di-(C₁-C₆)alkylamino, -C(=O)NH-(C₁-C₆)alkyl,(C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrile, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl,-(C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, and the radicals set forth in the definition of R⁶;
R¹⁰ is NHCR¹², NHCH₂R¹², NHSO₂R¹² or one of the radicals set forth in any of the definitions of R⁶, R⁸ and R⁹;
R¹¹ is oximino (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆)alkyl; and
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, and (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached.

Other examples of NK-1 receptor antagonists that can be used in the methods and pharmaceutical compositions of this invention are compounds of the formula I, as defined above, with the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group. Such compounds are hereinafter referred to as "compounds of the formula Ia".

Other examples of NK-1 receptor antagonists that can be used in the methods and pharmaceutical compositions of this invention are compounds of the formula 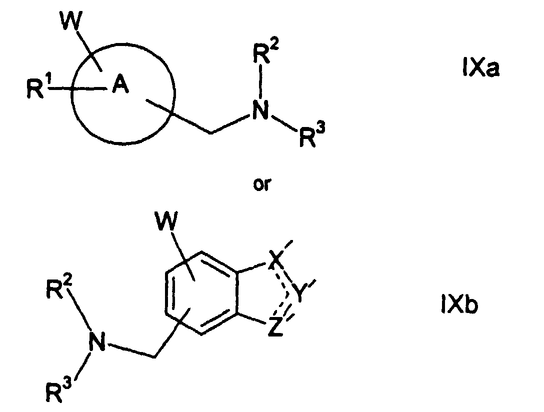 and their pharmaceutically acceptable salts, wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆)alkyl optionally substituted with from one to three fluorine atoms, - S(O)ᵥ-(C₁-C₆) alkyl wherein v is zero, one or two, halo, benzyloxy or (C₁-C₆)alkoxy optionally substituted with from one to three fluorine atoms;
R¹ is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazoiyl, isothiazolyl, imidazolyl, isoxazolyl, oxazolyl, pyridyl, pyrimidinyl, pyrazolyl or thiophenyl), wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
the dotted lines in formula Ib indicate that one of the X'-Y' and Y'-Z' bonds may optionally be a double bond;
X' is selected from =CH-, -CH₂-, -O-, -S- -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
Y' is selected from C=O, C=NR⁴, C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, =C(CF₃)-, =C(CH₂C₆H₅)-, -S- and SO₂, wherein the phenyl moieties of said =C(C₆H₅)- and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z' is selected from =CH-, -CH₂-, =N-, -NH-, -S-, -N(R⁴)-, =C(C₆H₅)-, -CH(C₆H₅)-, =C[(C₁-C₆) alkyl- and -CH[(C₁-C₆)alkyl]-;
or X', Y' and Z', together with the two carbon atoms shared between the benzo ring and the X'Y'Z' ring, form a fused pyridine or pyrimidine ring;
R² is hydrogen or -CO₂(C₁-C₁₀)alkyl;
R³ is selected from 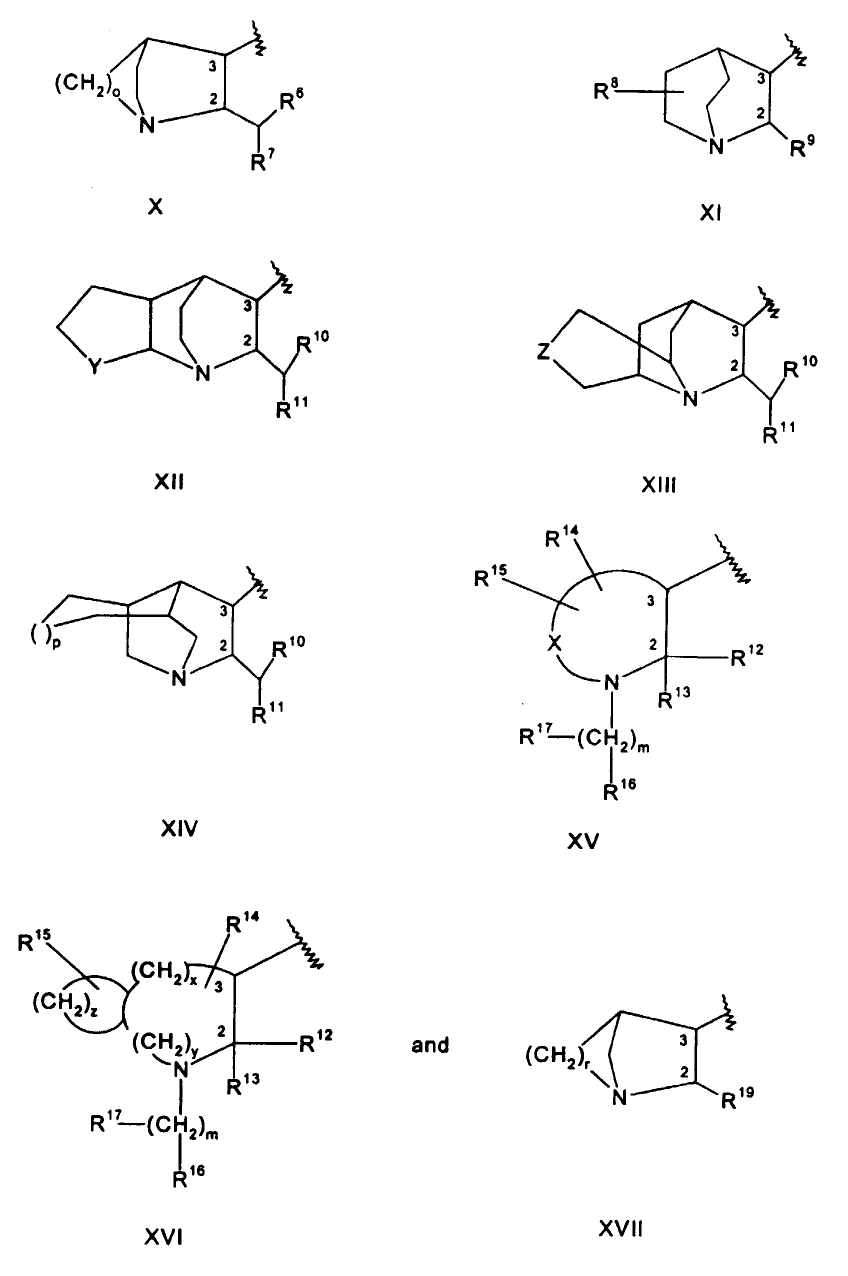
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁴ is (C₁-C₆) alkyl or phenyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 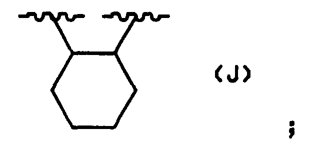
Z is oxygen, sulfur, amino, (C₁-C₃)alkylamino or (CH₂)ₙ wherein n is zero, one or two;
x is zero, one or two;
y is zero, one or two;
z is three, four or five; o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇) cycloalkyl wherein one of the carbon atoms may optionally be replaced by nitrogen, oxygen or sulfur, aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆) alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-, (C₁-C₆)alkyl-, di-(C₁-C₆)alkylamino, -C(=O)-NH-(C₁-C₆)alkyl, (C₁-C₆)-alkyl-C(=O)-NH-(C₁-C₆)alkyl, -NHC(=O)H and -NHC(=O)-(C₁-C₆)alkyl; and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to such point of attachment may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C(=O)-(C₁-C₆)alkyl-, and the radicals set forth in the definition of R¹²;
R¹⁶ is NHC(=O)R¹⁸, NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
with the proviso that (a) when m is 0, one of R¹⁶ and R¹⁷ is absent and the other is hydrogen, (b) when R³ is a group of the formula XVI, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ can not both be hydrogen, and (e) when R¹⁴ or R¹⁵ is attached to a carbon atom of X or (CH₂)_{y} that is adjacent to the ring nitrogen, then R¹⁴ or R¹⁵, respectively, must be a substituent wherein the point of attachment is a carbon atom.

The fused bicyclic nucleus of compounds of the formula IXb to which W and the -CH₂NR²R³ sidechain are attached may be, but is not limited to, one of the following groups: benzoxazolyl, benzthiazolyl, benzimidazolyl, benzisoxazolyl, benzoisothiazolyl, indazolyl, indolyl, isoquinolinyl, benzofuryl, benzothienyl, oxindolyl, benzoxazolinonyl, benzthiazolinonyl, benzimidazolinonyl, benzimidazoliniminyl, dihydrobenzothienyl-S,S-dioxide, benztriazolyl, benzthiadiazolyl, benzoxadiazolyl, and quinazolinyl.

Examples of acids that can be used to prepare pharmaceutically acceptable acid addition salts of eletriptan or basic NK-1 antagonists for use in this invention are those that which form non-toxic acid addition salts, *i*.*e*., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i*.*e*., 1,1□-methylene-bis-(2-hydroxy-3- naphthoate)]salts. The chemical bases that can be used as reagents to prepare the pharmaceutically acceptable base salts of NK-1 antagonists for use in this invention are those which form non-toxic base salts with the acidic compounds of formula I. Such non-toxic base salts include those derived from such pharmacologically acceptable cations as sodium, potassium calcium and magnesium, etc.

Other examples of NK-1 receptor antagonists that can be used in the method and pharmaceutical compositions of this invention are compounds of the formula 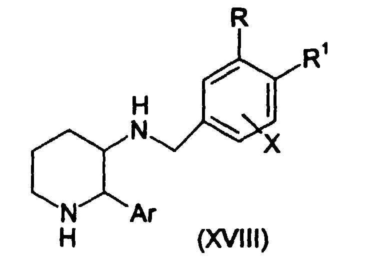 and their pharmaceutically acceptable salts, wherein
R is halo (C₁-C₈)alkyl, halo (C₂-C₈)alkenyl, halo (C₂-C₈)alkynyl or halo (C₁-C₈)alkyl substituted by hydroxy or (C₁-C₈)alkoxy; R¹ is hydrogen, halo or (C₁-C₆)alkoxy; or
R and R¹, together with the two carbon atoms shared between the benzene ring and the R and R¹, complete a fused (C₄-C₆)cycloalkyl wherein one carbon atom is optionally replaced by oxygen and wherein one or two of the carbon atoms are optionally substituted by up to five subtituents selected from halo, (C₁-C₆)alkyl and halo (C₁-C₆)alkyl;
X is (C₁-C₆)alkoxy, halo (C₁-C₆)alkoxy, phenoxy or halo; and
Ar is phenyl optionally substituents by halo.

Other examples of NK-1 receptor antagonists that can be used in the methods and pharmaceutical compositions of this invention are compounds of the formula 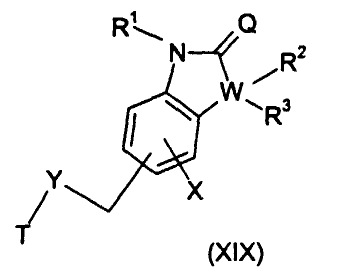 and their pharmaceutically acceptable salts, wherein
W is methylene, ethylene, propylene, vinylene, -CH₂-O-,-O-CH₂-, -CH₂-S- or -S-CH₂-;
R¹, R² and R³ are independently hydrogen, (C₁-C₃) alkyl, (C₁-C₃) alkoxy or halo (C₁-C₃) alkyl, provided that when W is methylene, both R² and R³ are not hydrogen;
X is halo, (C₁-C₃) alkoxy, (C₁-C₃) alkoxy or (C₁-C₃) alkenyl;
Y is imino or oxy;
Q is oxygen or sulfur; and
T is (2S,3S-2-diphenylmethylquinuclidin-3-yl,(2S,3S)-2-phenylpiperdin-3-yl or (2S,3S)-2-diphenylmethyl-1-azanorbornan-3-yl.

Other examples of NK-1 antagonists that can be used in the pharmaceutical compositions and methods of this inventions are the following compounds and their pharmaceutically acceptable salts: 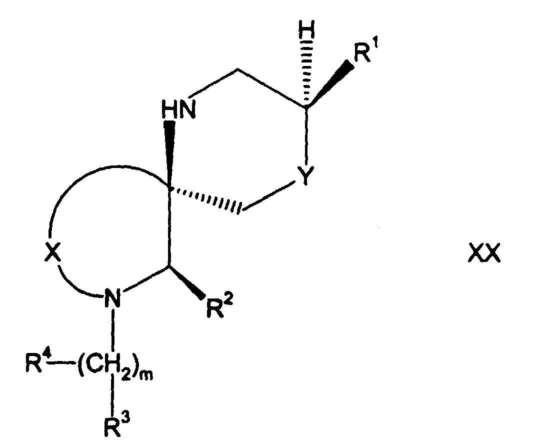
wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆)alkyl-C(=O)-NH-(C₁-C₆) alkyl, hydroxy(C₁-C₄)alkyl,-NHC(=O)H, -NHC(=O)-(C₁-C₆) alkyl, (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, (SO₂-(C₁-C₁₀)alkyl) ((C₁-C₁₀)alkyl)N wherein one or both of the alkyl moieties may optionally be substituted with from one to three fluorine atoms, -N(SO₂-(C₁-C₁₀)alkyl)₂ and (SO₂-aryl) ((C₁-C₁₀)alkyl)N; and wherein the aryl moieties of said -S(O)ᵥ-aryl, -O-aryl and (SO₂-aryl) ((C₁-C₁₀)alkyl)N are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 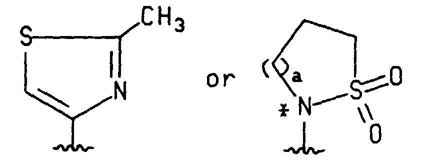 wherein a is 0, 1 or 2 and the asterisk represents a position meta to the point of attachment of R¹;
R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C-(C₁-C₆)alkyl-, di-(C₁-C₆)alkylamino, -C(=O)NH-(C₁-C₆)alkyl, (C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from NHC(=O)R⁸, NHCH₂R⁸, SO₂R⁸, AR⁵, CO₂H and the radicals set forth in the definitions of R², R⁶ and R⁷;
A is CH₂, nitrogen, oxygen, sulfur or carbonyl;
R⁸ is (C₁-C₆)alkyl, hydrogen, phenyl or phenyl (C₁-C₆)alkyl;
R⁴ is selected from oximino (=NOH) and the radicals set forth in the definitions of R², R⁶ and R⁷;
R⁵ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 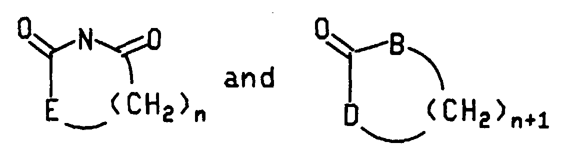 wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C-, (C₁-C₆)alkyl-C(=O)-(C₁-C₆)alky- and the radicals set forth in the definition of R²; and
Y is (CH₂)_{z} wherein z is zero or one;
with the proviso that: (a) when A is -(CH₂)- or carbonyl, R⁵ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; (c) when R⁶ or R⁷ is attached to a carbon atom of X that is adjacent to the ring nitrogen, then R⁶ or R⁷, respectively, must be a substituent wherein the point of attachment is a carbon atom;

Other examples of NK-1 receptor antagonists that can be used in the methods and pharmaceutical compositions of this invention include the following compounds and their pharmaceutically acceptable salts: 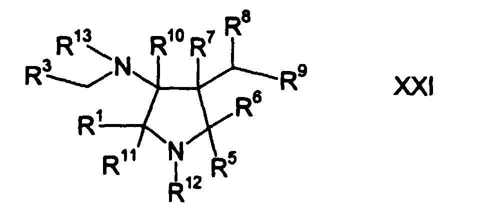
wherein R¹ is selected from hydrogen, (C₁-C₆) straight or branched alkyl, (C₃-C₇) cycloalkyl wherein one of the carbon atoms may optionally be replaced by nitrogen, oxygen or sulfur; aryl selected from phenyl, biphenyl, indanyl and naphthyl; heteroaryl selected from thienyl, fury), pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl optionally substituted with from one to three fluorine atoms, (C₁-C₆) alkoxy, amino, trihaloalkoxy (e.g., trifluoromethoxy), (C₁-C₆)alkylamino, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C-, (C₁-C₆)alkyl-O-, (C₁-C₆)alkyl-C(=O)-, (C₁-C₆)alkyl-C(=O)-, (C₁-C₆)alkyl-, di-(C₁-C₆)alkylamino, -C(=O)NH-(C₁-C₆)alkyl, (C₁-C₆)alkyl-C(=O)-NH-(C₁-C₆)alkyl-, -NHC(=O)H and -NHC(=O)-(C₁-C₆) alkyl; and wherein one of the phenyl moieties of said benzhydryl may optionally be replaced by naphthyl, thienyl, furyl or pyridyl;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl optionally substituted with from one to three fluorine atoms, (C₁-C₆) alkoxy, amino, phenyl, trihaloalkoxy (e.g., trifluoromethoxy), (C₁-C₆) alkylamino, -C(=O)-NH-(C₁-C₆)alkyl, (C₁-C₆)alkyl-C(=O)- -C-O-(C₁-C₆)alkyl, -C(=O)H, -CH₂OR¹³, NH(C₁-C₆)alkyl-, -NHC(=O)H, -NR²⁴C-(C₁-C₆)alkyl and -NHC(=O)-(C₁-C₆)alkyl;
one of R⁵ and R⁶ is hydrogen and the other is selected from hydroxymethyl, hydrogen, (C₁-C₃)alkyl, (C₁-C₈)acyloxy(C₁-C₃)alkyl, (C₁-C₈)alkoxymethyl and benzyloxymethyl;
R⁷ and R⁸ are independently selected from hydrogen, (C₁-C₃)alkyl and phenyl;
R⁹ is selected from methyl, hydroxymethyl, HC(=O)-, R¹⁴R¹⁵NCO₂CH₂-, R¹⁶OCO₂CH₂-, (C₁-C₄)alkyl-CO₂CH₂-, -CONR¹⁷R¹⁸, R¹⁷R¹⁸NCO₂-, R¹⁹OCO₂-, C₆H₅CH₂CO₂CH₂-, C₆H₅CO₂CH₂-, (C₁-C₄)alkyl-CH(OH)-, C₆H₅CH(OH)-, C₆H₅CH₂CH(OH)-, CH₂halo, R²⁰SO₂OCH₂, -CO₂R¹⁶ and R²¹CO₂-;
R¹⁰ and R¹¹ are independently selected from hydrogen, (C₁-C₃) alkyl and phenyl;
R¹² is hydrogen, benzyl or a group of the formula 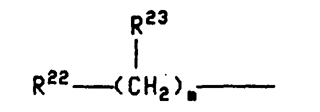
wherein m is an integer from zero to twelve, and any one of the carbon-carbon single bonds of (CH₂)ₘ may optionally be replaced by a carbon-carbon double or triple bond, and any one of the carbon atoms of (CH₂)ₘ may optionally be substituted with R²³ (as indicated by the slanted line to R²³ which intersects the horizontal line to (CH₂)ₘ in the above figure);
R¹³, R¹⁴, R¹⁵, R¹⁶, R¹⁷, R¹⁸, R¹⁹, R²⁰, R²¹ and R²⁴ are independently selected from hydrogen, (C₁-C₃)alkyl and phenyl;
R²² and R²³ are independently selected from hydrogen, hydroxy, halo, amino, carboxy, carboxy(C₁-C₆)alkyl, (C₁-C₆)alkylamino, di-(C₁-C₆)alkylamino, (C₁-C₆)alkoxy, (C₁-C₆)-alkyl-O-C(=O)-, (C₁-C₆)alkyl-O-C(=O)(C₁-C₆)alkyl, (C₁-C₆)alkyl-C(=O)- (C₁-C₆)alkyl-C(=O)-(C₁-C₆)alkyl-O-, (C₁-C₆)alkyl-C-, (C₁-C₆)-alkyl-C(=O)-(C₁-C₆)alkyl, (C₁-C₆) straight or branched alkyl, (C₃-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazol, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with one or two substituents independently selected from halo, nitro, (C₁-C₆)alkyl optionally substituted with from one to three fluorine atoms, (C₁-C₆)alkoxy optionally substituted with from one to three fluorine atoms, trifluoromethyl, amino, (C₁-C₆)-alkylamino, (C₁-C₆)alkyl-O-C(=O), (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C-(C₁-C₆)alkyl-, di-(C₁-C₆)alkylamino, C(=O)NH-(C₁-C₆)alkyl, (C₁-C₆)-alkyl-C(=O)-NH-(C₁-C₆)alkyl, -NHC(=O)H and -NHC(=O)-(C₁-C₆)alkyl; and wherein one of the phenyl moieties of said benzhydryl may optionally be replaced by naphthyl, thienyl, furyl or pyridyl;
or R⁹, together with the carbon to which it is attached, the nitrogen of the pyrrolidine ring, the carbon to which R⁷ is attached and the carbon to which R⁵ and R⁶ are attached form a second pyrrolidine ring; with the proviso that when R⁹, together with the carbon to which it is attached, the nitrogen of the pyrrolidine ring, the carbon to which R⁷ is attached and the carbon to which R⁵ and R⁶ are attached, form a second pyrrolidine ring (thus forming a bicyclic structure containing a bridgehead nitrogen), either R¹² is absent or R¹² is present and the nitrogen of the second pyrrolidine ring is positively charged.

Examples of specific NK-1 receptor antagonists that can be used in the methods and pharmaceutical compositions of this invention are the following compounds and their pharmaceutically acceptable salts:
(*2S,3S*)-3-[2-methoxy-5-(2-thiazolyl)benzyl]amino-2-phenylpiperidine;
(*2S,3S*)-3-(5-(2-imidazolyl)-2-methoxybenzyl]amino-2-phenylpiperidine;
(*2S,3S*)-3-(2-methoxy-5-(2-oxopyrrolidinyl)benzyl]amino-2-phenylpiperidine;
(*2S,3S*)-3-[2-methoxy-5-(4-methyl-2-thiazolyl)benzyl]-amino-2-phenylpiperidine;
(*2S,3S*)-3-(2-methoxy-5-(1,2,3-thiadiazol-4-yl)benzyl]amino-2-phenylpiperidine;
(*2S,3S*)-(6-methoxy-2-methyl-benzothiazol-5-ylmethyl)-(2-phenylpiperidin-3-yl)amine;
(*2S,3S*)-(5-(2,5-dimethyl-pyrrol-1-yl)-2-methoxybenzyl]-(2-phenylpiperidin-3-yl)amine;
(*2S,3S*)-3-[2-methoxy-5-(5-oxazolyl)benzyl]amino-2-phenylpiperidine;
(*2S,3S*)-(6-methoxy-2-phenyl-benzothiazol-5-ylmethyl)-(2-phenylpiperidin-3-yl)amine;
(*2S,3S*)-(6-methoxy-2-cyclopropyl-benzothiazol-5-ylmethyl)-(2-phenylpiperidin-3-yl)amine;
(*2S,3S*)-(6-methoxy-2-tert-butyl-benzothiazol-5-ylmethyl)-(2-phenylpiperidin-3-yl)amine;
(*2S,3S*)-(6-isopropoxyoxy-2-phenyl-benzothiazol-5-ylmethyl)-(2-phenylpiperidin-3-yl)amine;
(*2S,3S*)-(6-isopropoxyoxy-2-methyl-benzothiazol-5-ylmethyl)-(2-phenylpiperidin-3-yl)amine;
(*2S,3S*)-(6-trifluoromethoxy-2-methyl-benzothiazol-5-ylmethyl)-(2-phenylpiperidin-3-yl)amine;
(*2S,3S*)-(6-methoxy-2-methyl-benzoxazol-5-ylmethyl)-(2-phenylpiperidin-3-yl)amine;
(*1SR-2SR,3SR,4RS*)-3-(6-methoxy-3-methylbenzisoxazol-5-yl]methylamino-2-benzhydrylazanorbornane;
(*2S,3S*)-(2-methoxy-5-pyridin-2-ylbenzyl)-(2-phenylpiperidin-3-yl)amine;
(*2S,3S*)-(2-methoxy-5-pyrimidin-2-ylbenzyl)-(2-phenylpiperidin-3-yl)amine;
(*2S,3S*)-(2-methoxy-5-pyridin-3-ylbenzyl)-(2-phenylpiperidin-3-yl)amine;
(*2S,3S*)-(2-methoxy-5-(6-methylpyridin-2-yl)benzyl]-(2-phenylpiperidin-3-yl)amine;
(*2S,3S*)-[5-(3,5-dimethylpyrazol-1-yl)-2-methoxybenzyl]-(2-phenylpiperidin-3-yl)amine;
(*2S,3S*)-[2-methoxy-5-(3,4,5-trimethylpyrazol-1-yl)benzyl]-(2-phenylpiperidin-3-yl)amine;
(*2S,3S*)-(2-isopropoxy-5-(3,4,5-trimethytpyrazol-1-yl)benzyl)-(2-phenylpiperidin-3-yl)amine;
(*2S,3S*)-[5-(3,5-diisopropylpyrazol-1-yl)-2-methoxybenzyl]-(2-phenylpiperidin-3-yl)amine;
(*2S,3S*)-[5-(3,5-dimethylthiophen-2-yl)-2-methoxybenzyl]-(2-phenylpiperidin-3-yl)amine;
(*2S,3S*)-(6-methoxy-2,3-dimethyl-benzo[b]thiophen-7-ylmethyl)-(2-phenylpiperidin-3-yl)amine.
(*2S,3S*)-(6-methoxy-3-methyl-beno(d]isoxazol-5-ylmethyl)-(2-phenylpiperidin-3-yl)amine;
(*1SR,2SR,3SR,4RS*)-(2-benzhydryl-1-aza-bicydo[2.2.1)hept-3-yl)-6-methoxy-2-methyl-benzothiazol-5-ylmethyl)-amine;
(*2S,3S*)-(6-methoxy-benzoxazol-5-ylmethyl)-(2-phenyl-piperidin-3-yl)-amine;
(*2S,3S*)-(6-methoxy-benzothiazol-5-ylmethyl)-(2-phenyl-piperidin-3-yl)-amine;
(*2S,3S*)-5-methoxy-1-methyl-6-(2-phenylpiperidin-3-ylaminomethyl)-1,3-dihydro-indol-2-one;
(*2S,3S*)-6-methoxy-3-methyl-5-(2-phenylpiperidin-3-ylaminomethyl)-3H-benzoxazo1-2-one;
(*2S,3S*)-6-methoxy-3-methyl-5-(2-phenylpiperidin-3-ylaminomethyl)-3H-benzothiazol-2-one;
(*2S,3S*)-5-methoxy-1,3-dimethyl-6-(2-phenylpiperidin-3-ylaminomethyl)-1,3-dihydro-benzoimidazol-2-one;
(*2S,3S*)-(6-methoxy-3-methyl-3H-benzotriazol-5-ylmethyl)-(2-phenylpiperidin-3-yl)amine;
(*2S,3S*)-(2-methoxy-5-[1,2,3]thiadiazol-4-yl-benzyl)-(2-phenyl-1-azabicyclo[2.2.2]oct-3-yl)amine;
(*2S,3S*)-(2-methoxy-5-[1,2,3]thiadiazol-4-yl-benzyl)-(2-benzhydryl-1-azabicyclo[2.2.2)oct-3-yl)amine;
(*2S,3S*)-(6-methoxy-2-methyl-benzothiazol-5-ylmethyl)-(2-phenyl-1-azabicyclo[2.2.2]oct-3-yl)amine;
(*2S,3S*)-(6-methoxy-2-methyl-benzothiazol-5-ylmethyl)-(2-benzhydryl-1-azabicyclo[2.2.2)oct-3-yl)amine;
(*2S,3S*)-(2-methoxy-5-thiazol-2-yl-benzyl)-(2-benzhydryl-1-azabicyclo(2.2.2)oct-3-yl)amine;
(*2S,3S*)-(6-methoxy-2-methyl-benzothiazol-5-ylmethyl)-(2-phenyl-1-azabicyclo[2.2.1]hept-3-yl)amine;
(*2S,3S*)-(6-methoxy-2-methyl-benzothiazol-5-ylmethyl)-(2-benzhydryl-l-azabicyclo[2.2.1]hept-3-yl)amine;
(*2S,3S*)-(2-methoxy-5-[1,2,4]triazol-4-yl-benzyl)-(2-phenylpiperidin-3-yl)amine;
(*2S,3S*)-(2-methoxy-5-(1,2,4)triazol-1-yl-benzyl)-(2-phenylpiperidin-3-yl)amine;
(*2S,3S*)-(2-methoxy-5-thiazol-2-ylbenzyl)-(2-phenyl-decahydroquinolin-3-yl)amine;
(*2S,3S*)-(2-methoxy-5-thiazol-2-ylbenzyl)-(2-phenyl-octahydro-indol-3-yl)amine;
(*2S,3S*)-(2-methoxy-5-oxazol-4-ylbenzyl)-(2-phenylpiperidine-3-yl)amine;
(*2S,3S*)-(6-methoxy-2-(2-propyl)-benzothiazol-5-ylmethyl)-(2-phenylpiperidin-3-yl)amine;
(*1SR,2SR,3SR,4RS*)-(2-benzhydryl-1-azabicyclo[2.2.1]hept-3-yl)-(6-methoxy-2-phenylbenzothiazol-5-ylmethyl)amine;
(*1SR,2SR,3SR,4RS*)-(2-benzhydryl-1-azabicyclo[2.2.1]hept-3-yl)-(6-methoxy-2-cyclopropylbenzothiazol-5-ylmethyl)amine;
(*1SR,2SR,3SR,4RS*)-(2-benzhydryl-1-azabicyclo[2.2.1]hept-3-yl)-(6-methoxy-2-tert-butylbenzothiazol-5-ylmethyl)amine;
(*1SR,2SR,3SR,4RS*)-(2-benzhydryl-1-azabicyclo[2.2.1)hept-3-yl)-(6-methoxy-2-(2-propyl)benzothiazol-5-ylmethyl)amine;
(*1SR,2SR,3SR,4RS*)-(2-benzhydryi-1-azabicyclo[2.2.1]hept-3-yl)-(6-isopropoxyoxy-2-phenyl-benzothiazol-5-ylmethyl)amine;
(*1SR,2SR,3SR,4RS*)-(2-benzhydryl-1-azabicyclo[2.2.1]hept-3-yl)-(6-isopropoxyoxy-methyl-benzothiazol-5-ylmethyl)amine;
(*1SR,2SR,3SR,4RS*)-(2-benzhydryl-1-azabicyclo[2.2.1]hept-3-yl)-(6-trifluoromethoxy-2-methyl-benzothiazol-5-ylmethyl)amine;
(6-methoxy-1-oxa-2,3-diazainden-5-ylmethyl)-(2-phenyl-piperidin-3-yl)amine; and
(6-methoxy-2-methyl-1H-benzoimidazol-5-ylmethyl)-(2-phenylpiperidine-3-yl)amine.
(±)-[3R-[3α, 6α (R*)]]-3-phenyl-7-phenyl-1,8-diazaspiro[5.5]undecane;
(±)-[3R-[3α, 6α (R*)]]-3-(2-methoxyphenyl)-7-phenyl-1,8-diazaspiro[5.5]undecane;
(±)-[3R-[3α, 6α (R*)]]-3-(2-methoxy-5-trifluoromethoxy-phenyl)-7-phenyl-1,8-diazaspiro[5.5]undecane;
(±)-[3R-[3α, 6α (R*)]]-3-(5-chloro-2-methoxyphenyl)-7-phenyl-1,8-diazaspiro[5.5]undecane;
(±)-[3R-[3α, 6α (R*)]]-3-(5-isopropyl-2-methoxyphenyl)-7-phenyl-1,8-diazaspiro[5.5]undecane;
(±)-[3R-[3α, 6α (R*)]]-3-(5-tert.butyl-2-methoxyphenyl)-7-phenyl-1,8-diazaspiro[5.5]undecane;
(±)-[3R-[3α, 6α (R*)]]-3-(2-methoxy-5-(N-methyl-N-methylsulfonylaminophenyl)-7-phenyl-1,8-diazaspiro[5.5]-undecane;
(±)-[3R-[3α, 6α (R*)]]-3-(2-iodophenyl)-7-phenyl-1,8-diazaspiro[5.5]undecane;
(±)-[3R-[3α, 6α (R*)]]-3-(2-methoxy-4-methylphenyl)-7-phenyl-1,8-diazaspiro[5.5]undecane;
(±)-[3R-[3α, 6α (R*)]]-3-(2-isopropoxyphenyl)-7-phenyl-1,8-diazaspiro[5.5]-undecane;
(±)-[3R-[3α, 6α (R*)]]-3-(2-difluoromethoxy-5-trifluoromethoxyphenyl)-7-phenyl-1,8-diazaspiro[5.5]undecane;
(±)-[3R-[3α, 5α (R*)]]-3-(2-methoxyphenyl)-6-phenyl-1,7-diazaspiro[4.5]decane;
(±)-[3R-[3α, 5α (R*)]]-3-(2-methoxy-5-trifluoromethoxyphenyl)-6-phenyl-1,7-diazaspiro[4.5]decane;
(±)-[3R-[3α, 5α (R*)]]-3-(5-chloro-2-methoxyphenyl)-6-phenyl-1,7-diazaspiro[4.5]decane;
(±)-[3R-[3α, 5α (R*)]]-3-(5-isopropyl-2-methoxyphenyl)-6-phenyl-1,7-diazaspiro[4.5]decane;
(±)-[3R-[3α, 5α (R*)]]-3-(5-tert.butyl-2-methoxyphenyl)-6-phenyl-1,7-diazaspiro[4.5]decane;
(*2S, 3S*)-3-(2-Fluoro-5-(trifluoromethyl)benzyl)amino-2-phenylpiperidine;
(*2S, 3S*)-3-(2-Chloro-5-(trifluoromethyl)benzyl)amino-2-phenylpiperidine;
(*2S, 3S*)-3-(2-Methoxy-5-(trifluoromethyl)benzyl)amino-2-phenylpiperidine;
(*2S, 3S*)-3-(2-Phenoxy-5-(trifluoromethyl)benzylamino-2-phenylpiperidine;
(*2S, 3S*)-3-(5-(1,1-Difluoroethyl)-2-(trifluoromethoxy)benzyl)amino-2-phenylpiperidine;
(*2S,3S*)-3-(5-(1,1-Difluoroethyl)-2-methoxybenzyl)amino-2-phenylpiperidine;
(*2S,3S*)-3-(2-Methoxy-5-(2,2,2-trifluoroethyl)benzyl)amino-2-phenylpiperidine;
(*2S, 3S*)-3-(2-Methoxy-5-(1-(trifluoromethyl)ethyl)benzyl)amino-2-phenylpiperidine;
(*2S,,3S*)-3-[5-(1,1-dimethyl-4,4,4-trifluoro-2-butynyl)-2-methoxybenzyl)amino-2-phenyl piperidine;
(*2S,3S*)-3-[5-(1,1-Dimethyl-2,2,2-trifluoroethyl)-2-methoxybenzylamino]-2-phenylpiperidine;
(*2S,3S*)-3-(2,4-Dimethoxy-5-(2,2,2-trifluoroethyl)benzyl)amino-2-phenylpiperidine;
(*2S,3S*)-3-[5-[(1-Chloro-1-(trifluoromethyl)ethyl]-2-methoxybenzylamino]-2-phenylpiperidine;
(*2S,3S*)-2-Phenyl-3-(5-(2,2,2-trifluoro-1-(trifluoromethyl)ethyl)-2-methoxybenzyl)aminopiperidine;
(*2S,3S*)-2-Phenyl-3-(5-(2,2,2-trifluoro-1-(trifluoromethyl)ethyl)-2-methoxybenzyl)aminopiperidine;
(*2S,3S*)-2-Phenyl-3-(5-(1,2,2,2-tetrafluoro-1-(trifluoromethyl)ethyl)-2-methoxybenzyl)aminopiperidine;
(*2S,3S*)-3-(2-Methoxy-5-(1,1,2,2,2-pentafluoroethyl)benzyl)amino-2-phenylpiperidine;
(*2S,3S*)-2-Phenyl-3-(5-(2,2,2-trifluoro-1-methyl-1-(trifluoromethyl)ethyl)-2-methoxybenzyl)aminopiperidine;
(*2S,3S*)-3-[5-[2,2-Difluoro-1-(trifluoromethyl)ethenyl]-2-methoxybenzyl]amino-2-phenylpiperidine;
(*2S,3S*)-3-(2-Methoxy-5-(2,2,2-trifluoro-1-hydroxy-1-(trifluoromethyl)ethyl)benzyl)amino-2-phenylpiperidine;
(*2S,3S*)-3-[5-Methoxy-1-(trifluoromethyl)indan-6-yl)methylamino]-2-phenylpiperidine;
(*2S,3S*)-3-((6-Methoxy-1-(trifluoromethyl)-1,2,3,4-tetrahydronaphthalen-7-yl)methyl)amino-2-phenylpiperidinee;
(*2S,3S*)-3-((2,2-Difluoro-6-methoxy-1,2,3,4-tetrahydronaphthalen-7-yl)methyl)amino-2-phenylpiperidine;
(*2S,3S*)-3-(6-methoxy-1,3,3-trimethyloxindol-5-yl)methylamino-2-phenylpiperidine;
(2S,3S)-3-(6-methoxy-1-methyl-2-oxo-1,2,3,4-tetrahydroquinolin-7-yl)methylamino-2-phenylpiperidine;
(2S,3S)-3-(6-isopropoxy-1-methyl-2-oxo-1,2,3,4-tetrahydroquinolin-7-yl)methylamino-2-phenylpiperidine;
(2S,3S)-3-(1-isopropyl-6-methoxy-2-oxo-1,2,3,4-tetrahydroquinolin-7-yl)methylamino-2-phenylpiperidine;
(2S,3S)-3-[(6-methoxy-1-methyl-2-thioxo-1,2,3,4-tetrahydroquinolin-7-yl)methyl]amino-2-phenylpiperidine dihydrochloride;
(2S,3S)-3-[(7-methoxy-1-methyl-2-oxo-1,2,3,4-tetrahydroquinolin-6-yl)methyl]amino-2-phenylpiperidine dihydrochloride;
(2S,3S)-3-[(6-methoxy-1-methyl-2-oxo-4H-3,1-benzothiazin-7-yl)methyl]amino-2-phenylpiperidine dihydrochloride.
(2S,3S)-3-[(6-methoxy-1-methyl-2-oxo-4H-3,1-benzothiazin-7-yl)methyl]amino-2-phenylpiperidine dihydrochloride;
(2S, 3S, 4R)-2-diphenylmethyl-3-[(2-methoxy-4,5-dimethylphenyl)methylamino]-4-(2-hydroxyethyl)pyrrolidine;
(2SR, 3SR, 4RS)-2-diphenylmethyl-3-[(2-methoxy-4,5-dimethylphenyl)methylamino]-4-(2-hydroxyethyl)pyrrolidine;
(2SR, 3SR, 4RS)-2-diphenylmethyl-3-[(2-methoxy-5-(methylethyl)phenyl)methylamino]-4-(carbomethoxymethyl)- pyrrolidine;
(2SR, 3SR, 4RS)-2-diphenylmethyl-3-[(2-methoxy-5-(methylethyl)phenyl)methylamino]-4-(carboxymethyl)-pyrrolidine;
(2SR, 3SR, 4RS)-2-diphenylmethyl-3-[(2-methoxy-5-(methylethyl)phenyl)methylamino]-4-(2-dimethylamino-carbamoylethyl)pyrrolidine;
(2SR, 3SR, 4RS)-2-diphenylmethyl-3-[(2-trifluoromethoxphenyl)methylamino]-4-(2-hydroxyethyl)-pyrrolidine;
(2S, 3S, 4R)-2-diphenylmethyl-3-[(2-methoxy-5-(1,1-dimethylethyl)phenyl)methylamino]-4-(2-hydroxyethyl)- pyrrolidine;
(2SR, 3SR, 4RS)-2-diphenylmethyl-3-[(2-methoxy-5-(1,1-dimethylethyl)phenyl)methylamino]-4-(2-methoxyethyl)- pyrrolidine;
(2S, 3S, 4R)-2-diphenylmethyl-3-[(2-methoxy-5-methylethyl)phenyl)methylamino]-4-(2-hydroxyethyl)- pyrrolidine;
(2SR, 3SR, 4RS)-2-diphenylmethyl-3-[(2-methoxy-5-methylethyl)phenyl)methylamino]-4-(2-methoxyethyl)- pyrrolidine;
(2SR, 3SR, 4RS)-2-diphenylmethyl-3-[(2-methyl-5-(1,1-dimethylethyl)phenyl)methylamino]-4-(2-hydroxyethyl)- pyrrolidine;
(1SR, 2SR, 3SR, 4RS)-1-aza-2-diphenylmethyl-3-[(2-methoxy-4,5-dimethylphenyl)methylamino]-bicyclo[2.2.1]- heptane;
(1SR, 2SR, 3SR, 4RS)-1-aza-2-diphenylmethyl-3-[(2-methoxyphenyl)methylamino]bicyclo[2.2.1]heptane;
(1SR, 2SR, 3SR, 4RS)-1-aza-2-diphenylmethyl-3-[(2-methoxy-5-(1,1-dimethylethyl)phenyl)methylamino]bicyclo- [2.2.1]heptane;
(1SR, 2SR, 3SR, 4RS)-1-aza-2-diphenylmethyl-3-[(2-methoxy-5-trifluoromethoxyphenyl)methylamino]bicyclo- [2.2.1]heptane;
(1SR, 2SR, 3SR 4RS)-1-aza-2-diphenylmethyl-3-[(2-methoxy-5-(1-methylethyl)phenyl)methylamino]bicyclo- [2.2.1]heptane;
(1SR, 2SR, 3SR, 4RS)-1-aza-2-diphenylmethyl-3-[(2-methoxy-5-propylphenyl)methylamino]bicyclo[2.2.1]heptane;
(1SR, 2SR, 3SR, 4RS)-1-aza-2-diphenylmethyl-3-[(2-methoxy-5-(1-methylpropyl)phenyl)methylamino]bicyclo- [2.2.1]heptane;
(1SR, 2SR, 3SR, 4RS)-1-aza-2-phenyl-3-[(2-methoxyphenyl)methylamino]bicyclo[2.2.1]heptane;
(1SR, 2SR, 3RS, 4RS)-1-aza-2-phenyl-3-[(2-methoxy-5-trifluoromethoxyphenyl)methylamino]bicyclo[2.2.1]heptane;
(2SR, 3SR, 4RS)-N-1-phenylmethyl-2-diphenylmethyl-3-[(2-methoxypheny)methylamino]-4-(2-hydroxyethyl)- pyrrolidine;
(2SR, 3SR, 4RS)-2-diphenylmethyl-3-[(2-methoxy-phenyl)methylamino]-4-(2-hydroxyethyl)pyrrolidine;
(2SR, 3SR, 4RS)-2-diphenylmethyl-3-[(2-methoxy-5-(1,1-dimethylethyl)phenyl)methylamino]-4-(2-hydroxyethyl)- pyrrolidine;
(2SR, 3SR, 4RS)-diphenylmethyl-3-[(2-methoxy-5-trifluoromethoxyphenyl)methylamino]-4-(2-hydroxyethyl)- pyrrolidine;
(2SR, 3SR, 4RS)-2-diphenylmethyl-3-[(2-methoxy-5-(1-methylethyl)phenyl)methylamino]-4-(2-hydroxyethyl)- pyrrolidine;
(2SR, 3SR, 4RS)-2-diphenylmethyl-3-[(2-methoxy-5-propylphenyl)methylamino]-4-(2-hydroxyethyl)pyrrolidine;
(2SR, 3SR, 4RS)-2-diphenylmethyl-3-[(2-methoxy-5-(1-methyl-1-propyl)phenyl)methylamino]-4-(2-hydroxy-ethyl)pyrrolidine;
(2SR, 3SR, 4RS)-2-diphenylmethyl-3-[(2-trifluoromethoxy-5-(1,1-dimethylethyl)phenyl)methylamino]-4-(2-hydroxyethyl)pyrrolidine;
(2SR, 3SR, 4RS)-2-diphenylmethyl-3-[(2-methoxy-5-chlorophenyl)methylamino]-4-(2-hydroxyethyl)pyrrolidine;
(2SR, 3SR, 4RS)-2-phenyl-3-[(2-methoxyphenyl)methyl-amino]-4-(2-hydroxyethyl)pyrrolidine;
(2SR, 3SR, 4RS)-2-phenyl-3-[(2-methoxy-5-(1,1-dimethylethyl)phenyl)methylamino]-4-(2-hydroxy-ethyl)pyrrolidine;
(2SR, 3SR, 4RS)-2-phenyl-3-[(2-methoxy-5-trifluoromethoxyphenyl)methylamino]-4-(2-hydroxy-ethyl)pyrrolidine;
6-Methoxy-1,3,3-trimethyl-5-[(2-phenyl-piperidin-3-ylamino)-methyl]-3-dihydro-indol-2-one;
6-Methoxy-1,methyl-7-[(2-phenyl-piperidin-3-ylamino)-methyl]-,3-4-dihydro-1H-quindol-2-one;
6-Methoxy-1,2-dimethyl-1,2,3,4-terrahydro-quinolin-7-ylmethyl)-(2-phenyl-piperidin-3-yl)-amine;
6-Isopopoxy-1-methyl-7-[(2-phenyl-piperidin-3-ylamino)-methyl]-3,4-dihydro-1H-quinolin-2-one;
7-Methoxy-1-methyl-6-[(2-phenyl-piperidin-3-ylamino)-methyl]-3,4-dihydro-1H-quinolin-2-one;
6-Methoxy-1-methyl-7-[(2-phenyl-piperidin-3-ylamino)-methyl]-1,4-dihydro-benzo[d][1,3]thiazin-2-one;
6-Methoxy-7-[(2-phenyl-piperidin-3-ylamino)-methyl]1-(2,2,2-trifluoroethyl)-3,4-dihydro-1H-quinolin-2-one;
6-Isopopoxy-1-methyl-7-[(2-phenyl-piperidin-3-ylamino)-methyl]-3,4-dihydro-1H-quinolin-2-thione; and
6-Methoxy-1,3-dimethyl-7-[(2-phenyl-piperidin-3-ylamino)-methyl]-3,3-dihydro-1H-quinolin-2-one.

Preferred embodiments of this invention relate to the above pharmaceutical compositions for the treatment or prevention of migraine, and the above methods of treating or preventing migraine, wherein the NK-1 receptor antagonist, or pharmaceutically acceptable salt thereof, is selected from the following compounds and their pharmaceutically acceptable salts:
(6-Methoxy-3-trifluoromethyl-benzo[d]isoxazol-5-ylmethyl)-(2-phenyl-piperidin-3-yl)amine;
6-Methoxy-1-methyl-7-[(2-phenyl-1-propyl-piperidin-3-ylamino)-methyl]-3,4-dihydro-1H-quinolin-2-one;
6-Methoxy-1-methyl-7-{[1-(5-oxo-2,5-dihydro-1H-[1,2,4]triazol-3-ylmethyl)-2-phenylpiperidin-3-ylamino]-methyl}-3,4-dihydro-1H-quinolin-2-one;
3-(2-Methoxy-5-trifluoromethoxy-phenyl)-6-phenyl-1,7-diaza-spiro[4.5]decane;
6-Methoxy-1-methyl-7-[(2-phenyl-piperidin-3-ylamino)-methyl]-3,4-dihydro-1H-quinolin-2-one;
[2-Methoxy-5-(2,2,2-trifluoro-1-trifluoromethyl-ethyl)-benzyl]-(2-phenyl-piperidin-3-yl)amine;
[5-(1,1-Dimethy-prop-2-ynyl)-2-methoxy-benzyl]-(2-phenyl-piperidin-3-yl)-amine;
7-Methoxy-1-methyl-6-[(2-phenyl-piperidin-3-ylamino)-methyl]-3,4-dihydro-1H-quinolin-2-one;
[2-Methoxy-5-(2,2,2-trifluoro-1,1-dimethyl-ethyl)-benzyl]-(2-phenyl-pipendin-3-yl)amine;
(7-Methoxy-4-methyl-3,4-dihydro-2H-benzo[1,4]oxazin-6-ylmethyl)-(2-phenylpiperidin-3-yl)-amine;
[2-Methoxy-5-(1-methyl-1-trifluoromethyl-prop-2-ynyl)-benzyl]-(2-phenyl-piperidin-3-yl)-amine;
(6-Methoxy-1-methyl-1-trifluoromethyl-isochroman-7-ylmethyl)-(2-phenyl-piperidin-3-yl)-amine;
2-{3-[(2-Benzhydryl-1-aza-bicyclo[2.2.2]oct-3-ylamino)-methyl]-4- methoxy-phenyl}-2-methyl-propan-1-ol;
3-(3,5-Bis-trifluoromethyl-benzyloxy)-2-phenyl-piperidine;
5-[2-(3,5-Bis-trifluoromethyl-benzyloxy)-3-phenyl-morpholin-4-ylmethyl]-2,4-dihydro[1,2,4]triazol-3-one;
(*2S, 3S*)-3-(2-Methoxy-5-(trifluoromethoxy)benzyl)amino-2-phenylpiperidine;
(2S, 3S)-N-(5-isopropy-2-methoxyphenyl)methyl-2-diphenylmethyl-1-azabicyclo[2.2.2]octan-3-amine;
(2S, 3S)-N-(5-tert-butyl-2-methoxyphenyl)methyl-2-diphenylmethyl-1-azabicyclo[2.2.2]octane-3-amine;
(2S, 3S)-N-(5-ethyl-2-methoxyphenyl)methyl-2-diphenylmethyl-1-azabicyclo[2.2.2]octan-3-amine; and
(2S,3S)-N-(5-n-propyl-2-methoxyphenyl)methyl-2-diphenylmethyl-1-azabicydo[2.2.2]octane-3-amine.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined as above.

The term "one or more substituents, as used herein, includes from one to the maximum number of substituents possible based on the number of available bonding sites.

The term "antimigraine effective amount", as used herein, refers to an amount that is effective in treating migraine.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The pharmaceutical compositions and methods of this invention comprise, or comprise administering NK-1 receptor antagonists of the formulas I through XXI, which may have chiral centers and therefore exist in different enantiomeric forms. This invention includes methods and pharmaceutical compositions, as described above, wherein the NK-1 receptor antagonists that are employed are optical isomers tautomers or stereoisomers of the compounds of formulas I through XXI that are defined above, or mixtures thereof.

This present invention also relates to pharmaceutical compositions and methods comprising, or comprising administering, pharmaceutically acceptable acid addition salts of eletriptan and an NK-1 antagonist. The possible acids that are used to prepare the pharmaceutically acceptable acid addition salts of the basic NK-1 receptor antagonists and antidepressant and antianxiety agents employed in the compositions and methods of this invention are those which form non-toxic acid addition salts, *i*.*e*., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i*.*e*., 1,1'-methylene-bis-(2-hydroxy-3- naphthoate)]salts, with such compounds.

This invention also relates to pharmaceutical compositions and methods comprising, or comprising administering, pharmaceutically acceptable base addition salts of acidic NK-1 receptor antagonists and antidepressant and antianxiety agents. The chemical bases that may be used as reagents to prepare pharmaceutically acceptable base salts of the NK-1 receptor antagonists and antidepressant and antianxiety agents that are employed in the compositions and methods of this invention are those that form non-toxic base salts with such compounds. Such non-toxic base salts include, but are not limited to those derived from such pharmacologically acceptable cations such as alkali metal cations (*e*.*g*., potassium and sodium) and alkaline earth metal cations (*e*.*g*., calcium and magnesium), ammonium or water-soluble amine addition salts such as N-methylglucamine(meglumine), and the lower alkanolammonium and other base salts of pharmaceutically acceptable organic amines.

The subject invention also relates to pharmaceutical compositions and methods of treatment that employ isotopically-labeled compounds that are identical to those recited in formulas I through XXI, or to other NK-1 receptor antagonists, but for the fact that one or more atoms are replaced by an atom having an atomic mass or mass number different from the atomic mass or mass number usually found in nature. Examples of isotopes that can be incorporated into the NK-1 receptor antagonists that are employed in the pharmaceutical compositions and methods of the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. The NK-1 receptor antagonists employed in the pharmaceutical compositions and methods of the present invention, prodrugs thereof, and pharmaceutically acceptable salts of said compounds or of said prodrugs which contain the aforementioned isotopes and/or other isotopes are within the scope of this invention. Certain isotopically-labeled NK-1 receptor antagonists, for example, those into which radioactive isotopes such as ³H and ¹⁴C are incorporated, are useful in drug and/or substrate tissue distribution assays. Tritiated, *i*.*e*., ³H, and carbon-14, *i*.*e*., ¹⁴C, isotopes are particularly preferred for their ease of preparation and detectability. Further, substitution with heavier isotopes such as deuterium, *i*.*e*., ²H, can afford certain therapeutic advantages resulting from greater metabolic stability, for example increased *in vivo* half-life or reduced dosage requirements and, hence, may be preferred in some circumstances.

### Detailed Description Of The Invention

The following references refer, collectively, to quinuclidine, piperidine, ethylene diamine, pyrrolidine and azanorbornane derivatives and related compounds that exhibit activity as NK-1 receptor antagonists and that can be used, in combination with eletripan, in the pharmaceutical compositions and methods of this invention, and to methods of preparing the same: United States Patent 5,162,339, which issued on November 11, 1992; United States Patent 5,232,929, which issued on August 3, 1993; World Patent Application WO 92/20676, published November 26, 1992; World Patent Application WO 93/00331, published January 7, 1993; World Patent Application WO 92/21677, published December 10, 1992; World Patent Application WO 93/00330, published January 7, 1993; World Patent Application WO 93/06099, published April 1, 1993; World Patent Application WO 93/10073, published May 27, 1993; World Patent Application WO 92/06079, published April 16, 1992; World Patent Application WO 92/12151, published July 23, 1992; World Patent Application WO 92/15585, published September 17, 1992; World Patent Application WO 93/10073, published May 27, 1993; World Patent Application WO 93/19064, published September 30, 1993; World Patent Application WO 94/08997, published April 28, 1994; World Patent Application WO 94/04496, published March 3, 1994; World Patent Application WO 95/07908, published March 3, 1995; World Patent Application WO 95/16679, published June 22, 1995; World Patent Application WO 94/20500, published September 15, 1994; World Patent Application WO 94/13663, published June 23, 1994; World Patent Application WO 97/08144, published March 6, 1997; World Patent Application WO 97/03066, published January 30, 1997; World Patent Application WO 99/25714, published May 27, 1999; United States Patent Application 988,653, filed December 10, 1992; United States Patent Application 026,382, filed March 4, 1993; United States Patent Application 123,306, filed September 17, 1993, and United States Patent Application 072,629, filed June 4, 1993. All of the foregoing World Patent Applications designate the United States. The foregoing patents and patent applications are incorporated herein by reference in their entirety.

NK-1 receptor antagonists of the formula I can be prepared as described in the following patents and patent applications, all of which are referred to above and incorporated herein by reference in their entirety WO 93/00331, WO 92/21677, WO 92/15585, WO 92/01688, WO 93/06099, WO 91/18899, United States Patent 5,162,339,and United States Patent 5,232,929. NK-1 receptor antagonists of the formula Ia (*i*.*e*., compounds defined identically to compounds of the formula I, but having the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group) can be prepared as described in WO 93/00331.

NK-1 receptor antagonists of the formula IXa and IXb can be prepared as described in World Patent Application WO 94/13663, published June 23, 1994.

NK-1 receptor antagonists of the formula XVIII can be prepared as described in World Patent Application WO 97/08144, published March 6, 1997.

NK-1 receptor antagonists of the formula XIX can be prepared as described in World Patent Application WO 97/03066, published January 30, 1997 and World Patent Application WO 99/25714, published May 27, 1999.

NK-1 receptor antagonists of the formula XX can be prepared as described in World Patent Application WO 94/20500, published September 15, 1994.

NK-1 receptor antagonists of the formula XXI can be prepared as described in World Patent Application WO 93/00330, published January 7, 1993.

Other NK-1 receptor antagonists that can be used, together with eletriptan or a pharmaceutically acceptable salt of eletriptan, in the pharmaceutical compositions and methods of this invention are those compounds and pharmaceutically acceptable salts described in the following references: European Patent Application EP 499,313, published August 19, 1992; European Patent Application EP 520,555, published December 30, 1992; PCT Patent Application WO 95/16679, published June 22, 1995; European Patent Application EP 522,808, published January 13, 1993, European Patent Application EP 528,495, published February 24, 1993, PCT Patent Application WO 93/14084, published July 22, 1993, PCT Patent Application WO 93/01169, published January 21, 1993, PCT Patent Application WO 93/01165, published January 21, 1993, PCT Patent Application WO 93/01159, published January 21, 1993, PCT Patent Application WO 92/20661, published November 26, 1992, European Patent Application EP 517,589, published December 12, 1992, European Patent Application EP 428,434, published May 22, 1991, and European Patent Application EP 360,390, published March 28, 1990. All of the foregoing World Patent Applications designate the United States. The foregoing patents and patent applications are incorporated herein by reference in the entirety.

Eletriptan and methods of making it are referred to in United States Patent 5,545,644, which issued on August 13, 1996. The hydrobromide salt of eletriptan, and methods of preparing it are referred to in Europe Patent application EP 776,323, which was published on June 4, 1997. A method of treating emesis using eletriptan is referred to in United States Patent 5,618,834, which issued on April 8, 1997. Compositions containing eletriptan hemisulfate and caffeine and their use in the treatment of migraine and other headaches are referred to in World Patent Application PCT/EP98/04176 which was filed on July 1, 1998.

This invention relates both to methods of treating migraine in which the NK-1 receptor antagonist and eletriptan, or a pharmaceutically acceptable salt of eletriptan, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the NK-1 receptor antagonist will be administered to an adult human in an amount ranging from about 0.05 to about 1500 mg per day, in single or divided doses, preferably from about 5 to about 200 mg/day, and eletriptan or a pharmaceutically acceptable salt of eletriptan will be administered in an amount ranging from about 0.1 to about 800 mg per day, in single or divided doses, preferably from about 0.1 to about 400 mg/day when administered orally, buccally or parenterally and from about 100 µg to about 10 mg/day when administered as an aerosol formulation. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The NK-1 receptor antagonists, their pharmaceutically acceptable salts eletriptan and the pharmaceutically acceptable salts of eletriptan that are employed in the pharmaceutical compositions and methods of this invention are hereinafter also referred to as "therapeutic agent". The therapeutic agents can be administered via either the oral or parenteral route. Compositions containing both an NK-1 receptor antagonist and eletriptan or a pharmaceutically acceptable salt of eletriptan will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i*.*e*., they may be combined with various pharmaceutically acceptable inert carriers in the form of tablets, capsules, lozenges, froches, hard candies, suppositories, aqueous suspensions, injectable solutions, elixirs, syrups, and the like. Such carriers include solid diluents or fillers, sterile aqueous media and various non-toxic organic solvents, *etc*. Moreover, oral pharmaceutical compositions can be suitably sweetened and/or flavored. In general, the therapeutic compounds of this invention, when administered separately (*i*.*e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the NK-1 receptor antagonist and eletriptan may be formulated in a single pharmaceutical composition or alternatively in individual pharmaceutical compositions for simultaneous, separate or sequential use in accordance with the present invention.

Preferably, the compositions according to the present invention, which contain eletriptan and an NK-1 receptor antagonist, as well as the individual pharamaceutical compositions used to deliver any one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the therapeutic agents are mixed with a pharmaceutical carrier, *e*.*g*., conventional tableting ingredients such as corn starch, lactose, sucrose, sorbitol, talc, stearic acid, magnesium stearate, dicalcium phosphate or gums, and other pharmaceutical diluents, *e*.*g*., water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.05 to about 500 mg of each of the therapeutic agents of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration by injection include those comprising the therapeutic agent or agents in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.*, Tween™ 20, 40, 60, 80 or 85) and other sorbitans (*e*.*g*., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™ , Lipofundin ™ and Lipiphysan™. The therapeutic agents may be either dissolved in a pre-mixed emulsion composition or alternatively it may be dissolved in an oil (*e*.*g*., soybean oil, safflower oil, cottonseed oil, sesame oil, corn oil or almond oil) and an emulsion formed upon mixing with a phospholipid (*e*.*g*., eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include, solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising an NK-1 receptor antagonist and eletriptan, which process comprises bringing an NK-1 receptor antagonist and eletriptan into association with a pharmaceutically acceptable carrier or excipient.

When administered in combination, either as a single or as separate pharmaceutical composition(s), the NK-1 receptor antagonist and eletriptan are presented in a ratio which is consistent with the manifestation of the desired effect. A suitable dosage level for the NK-1 receptor antagonist is about 0.05 to 1500 mg per day, preferably about 5 to 200 mg per day. The compounds may be administered on a regimen of up to 6 times per day, preferably 1 to 4 times per day.

A suitable oral dosage level for the eletriptan is about 0.5 to 1500 mg per day, preferably about 20 to 200 mg per day. The compounds may be administered on a regimen of up to 6 times per day, preferably 1 to 4 times per day.

Other preferred embodiments include the delivery of the NK-1 receptor antagonist using an oral dosage form or by injection and the delivery of the eletriptan as a conventional tablet, liquid, elixir or suspension.

5-HT_{lD} agonists have a systemic mechanism of action. While the rate of headache recurrence with 5-HT_{1D} agonists is approximately 40% within a 24 hour period, the overall recurrence rate will decrease when a tachykinin antagonist and eletriptan are administered together in the treatment of migraine, since the combination will affect the migraine in two different ways. Firstly, the eletriptan will lessen the signals to the sensory nerves. Secondly, simultaneously, the NK-1 receptor antagonist will block inflammation around blood vessels in sensitive tissues such as the dura mater. Since the pathogenic circle in migraine is influenced by these two major mechanisms, the chances for headache relapse decrease. When a NK-1 receptor antagonist and eletriptan are both used in the treatment of migraine, both mechanisms will be suppressed and the duration of action in the treatment of migraine will therefore be increased.

It will be appreciated that the amount of the NK-1 receptor antagonist and eletriptan required for use in the treatment or prevention of migraine will vary not only with the particular compounds or compositions selected but also with the route of administration, the nature of the condition being treated, and the age and condition of the patient, and will ultimately be at the discretion of the patient's physician or pharmacist.

The activity of the compounds of the present invention, as substance P antagonists, is determined by their ability to inhibit the binding of substance P at its receptor sites in CHO-cells which reveal NK1 receptor or IM-9 cells employing radioactive ligands. The substance P antagonist activity of the herein described piperidine compounds is evaluated using the standard assay procedure described by M. A. Cascieri *et al*., as reported in The Journal of Immunology, 133, 3260 (1984). This method essentially involves determining the concentration of the individual compound required to reduce by 50% the amount of radiolabelled substance P ligands at their receptor sites in said isolated cow tissues or IM-9 cells, thereby affording characteristic IC₅₀ values for each compound tested. More specifically, inhibition of [³H]SP binding to human IM-9 cells by compounds is determined in assay buffer (50 mM Tris-HCl (ph 7.4), 1mM MnCl₂, 0.02% bovine serum albumin, bacitracin (40 µg/ml) leupeptin (4 µg/ml), chymostatin (2 µg/ml) and phosphoramidon (30 µg/ml). The reaction is initiated by the addition of cells to the assay buffer containing 0.56 nM [³H]SP and various concentrations of compounds (total volume; 0.5 ml) and allowed to incubate for 120 minutes at 4°C. Incubation is terminated by filtration onto GF/B filters (presoaked in 0.1% polyethylenimine for 2 hours). Nonspecific binding is defined as the radioactivity remaining in the presence of 1µM SP. The filters are placed into tubes and counted using a liquid scintillation counter.

Compounds and salts can be evaluated as antimigraine agents by testing the extent to which they mimic sumatriptan in contracting the dog isolated saphenous vein strip (P.P.A. Humphrey et al., Br. J. Pharmacol., 1988; 94: 1128.). This effect can be blocked by methiothepin, a known serotonin antagonist. Sumatriptan is known to be useful in the treatment of migraine and produces a selective increase in carotid vascular resistance in the anaesthetized dog. It has been suggested that this is the basis of its efficacy by Fenwick et al., Br. J. Pharmacol., 1989; 96: 83.

The following example illustrates pharmaceutical compositions according to the invention.

These formulations may be prepared with separate active ingredients or with a combination of active ingredients in one composition. In such combined preparations, the ratio of NK-1 antagonist to eletriptan will depend upon the choice of active ingredients.

### EXAMPLE 1A

### Tablets containing 10-100 mg of the NK-1 antagonist and 20-80 mg of eletriptan

| | Amount (mg) | | | | | | | | |
|---|---|---|---|---|---|---|---|---|---|
| NK-1 antagonist | 5.0 | 15.0 | 50.0 | 5.0 | 15.0 | 50.0 | 5.0 | 15.0 | 50.0 |
| Eletriptan | 20.0 | 20.0 | 20.0 | 40.0 | 40.0 | 40.0 | 80.0 | 80.0 | 80.0 |
| Microcrystalline cellulose | 20.0 | 20.0 | 20.0 | 20.0 | 20.0 | 20.0 | 20.0 | 20.0 | 20.0 |
| Modified food corn starch | 20.0 | 20.0 | 20.0 | 20.0 | 20.0 | 20.0 | 20.0 | 20.0 | 20.0 |
| Lactose | 52.5 | 47.5 | 34.5 | 29.5 | 29.5 | 29.5 | 29.5 | 29.5 | 29.5 |
| Magnesium Stearate | 0.5 | 0.5 | 0.5 | 0.5 | 0.5 | 0.5 | 0.5 | 0.5 | 0.5 |

### EXAMPLE 1B

### Tablets containing 20-300 mg of the NK-1 antagonist and 20-80 mg of eletriptan

| | Amount (mg) | | | | | | | | |
|---|---|---|---|---|---|---|---|---|---|
| NK-1 antagonist | 20.0 | 20.0 | 20.0 | 100.0 | 100.0 | 100.0 | 300.0 | 300.0 | 300.0 |
| Eletriptan | 20.0 | 40.0 | 80.0 | 20.0 | 40.0 | 80.0 | 20.0 | 40.0 | 80.0 |
| Microcrystalline cellulose | 20.0 | 20.0 | 20.0 | 20.0 | 20.0 | 20.0 | 20.0 | 20.0 | 20.0 |
| Modified food corn starch | 20.0 | 20.0 | 20.0 | 20.0 | 20.0 | 20.0 | 20.0 | 20.0 | 20.0 |
| Lactose | 52.5 | 47.5 | 34.5 | 29.5 | 29.5 | 29.5 | 29.5 | 29.5 | 29.5 |
| Magnesium Stearate | 0.5 | 0.5 | 0.5 | 0.5 | 0.5 | 0.5 | 0.5 | 0.5 | 0.5 |

The active ingredients, cellulose, lactose and a portion of the corn starch are mixed and granulated with 10% corn starch paste. The resulting granulation is sieved, dried and blended with the remainder of the corn starch and the magnesium stearate. The resulting granulation is then compressed into tablets containing 2.0 mg, 20.0 mg, 26.0 mg, 40.0 mg and 100 mg of the NK-1 receptor antagonist and 5.0 mg or 10.0 mg of eletriptan per tablet.

## Claims

1. A pharmaceutical composition for the treatment of migraine comprising: (a) eletriptan or a pharmaceutically acceptable salt of eletriptan; (b) a CNS-penetrant NK-1 receptor antagonist or a pharmaceutically acceptable salt thereof, and (c) a pharmaceutically acceptable carrier, wherein the amounts of the two active agents (a) and (b) in said composition are chosen so that the composition is effective in treating migraine.

2. A pharmaceutical composition according to claim 1, wherein the NK-1 receptor antagonist or pharmaceutically acceptable salt thereof is selected from compounds of the formula I, as defined below, and their pharmaceutically acceptable salts: 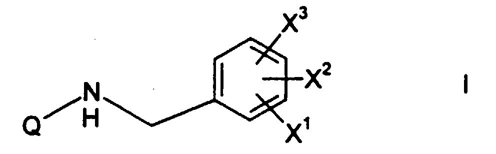
wherein X¹ is hydrogen, (C₁-C₁₀) alkoxy optionally substituted with from one to three flourine atoms or (C₁-C₁₀) alkyl optionally substituted with from one to three fluorine atoms;
X² and X³ are independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamnino, -C(=O)-NH-(C₁-C₆)alkyl, (C₁-C₆) alkyl-C(=O)-NH-(C₁-C₆) alkyl, hydroxy(C₁-C₄)alkyl, (C₁-C₄)alkoxy(C₁-C₄)alkyl, -NHC(=O)H and -NHC(=O)-(C₁-C₆) alkyl; and
Q is a group of the formula 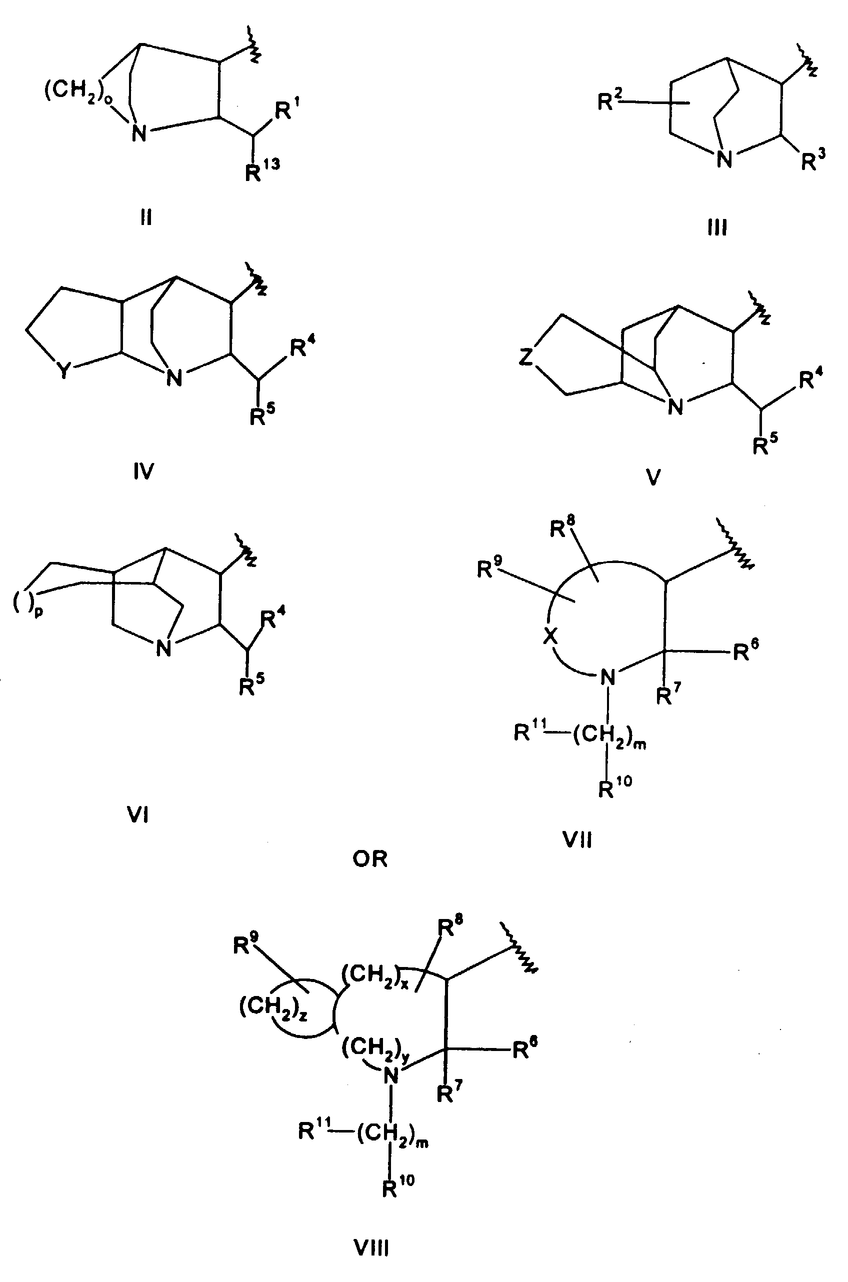 wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R¹³ is selected from (C₃-C₄) branched alkyl, (C₅-C₆) branched alkenyl, (C₅-C₇) cydoalkyl, and the radicals named in the definition of R¹;
R² is hydrogen or (C₁-C₆) alkyl;
R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 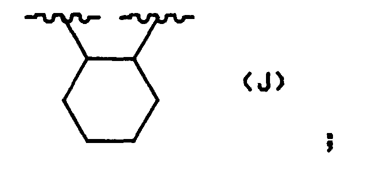
Z is oxygen, sulfur, amino, (C₁-C₃)alkylamino or (CH₂)ₙ, wherein n is zero, one or two;
o is two or three;
p is zero or one;
R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy(C₁C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆)alkyl-C(=O)-O-, (C₁-C₆)alky-C(=O)-(C₁-C₆)alkyl-O-, (C₁-C₆)alkyl-C(=O)-, (C₁C₆)alkyl-C(=O)-(C₁-C₆)alkyl-, di-(C₁-C₆)alkylamino, -C(=O)NH-(C₁-C₆)alkyl,(C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrile, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl,-(C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, and the radicals set forth in the definition of R⁶;
R¹⁰ is NHCR¹², NHCH₂R¹², NHSO₂R¹² or one of the radicals set forth in any of the definitions of R⁶, R⁸ and R⁹,
R¹¹ is oximano (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆) alkyl; and
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, and (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached.

3. A pharmaceutical composition according to claim 1, wherein the NK-1 receptor antagonist or pharmaceutically acceptable salt thereof is selected from compounds of the formula IXa or IXb, as defined below, and their pharmaceutically acceptable salts: 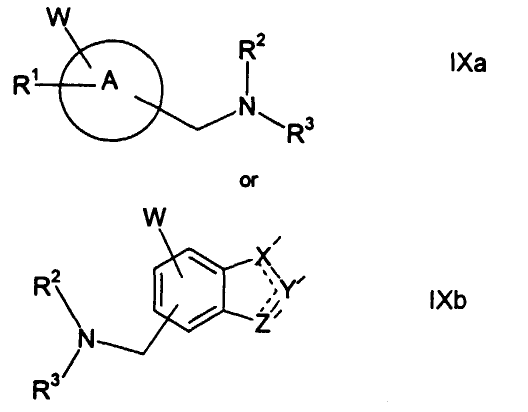 and their pharmaceutically acceptable salts, wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆)alkyl optionally substituted with from one to three fluorine atoms, - S(O)ᵥ-(C₁-C₆) alkyl wherein v is zero, one or two, halo, benzyloxy or (C₁-C₆)alkoxy optionally substituted with from one to three fluorine atoms;
R¹ is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, oxazolyl, pyridyl, pyrimidinyl, pyrazolyl or thiophenyl), wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
the dotted lines in formula Ib indicate that one of the X'-Y' and Y'-Z' bonds may optionally be a double bond;
X' is selected from =CH-, -CH₂-, -O-, -S-, -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
Y' is selected from C=O, C=NR⁴, C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, =C(CF₃)-, =C(CH₂C₆H₅)-, -S- and SO₂, wherein the phenyl moieties of said =C(C₆H₅)- and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z' is selected from =CH-, -CH₂-, =N-, -NH-, -S-, -N(R⁴)-, =C(C₆H₅)-, -CH(C₆H₅)-, =C[(C₁-C₆) alkyl]- and -CH[(C₁-C₆)alkyl]-;
or X', Y' and Z', together with the two carbon atoms shared between the benzo ring and the X'Y'Z ring, form a fused pyridine or pyrimidine ring;
R² is hydrogen or -CO₂(C₁-C₁₀)alkyl;
R³ is selected from 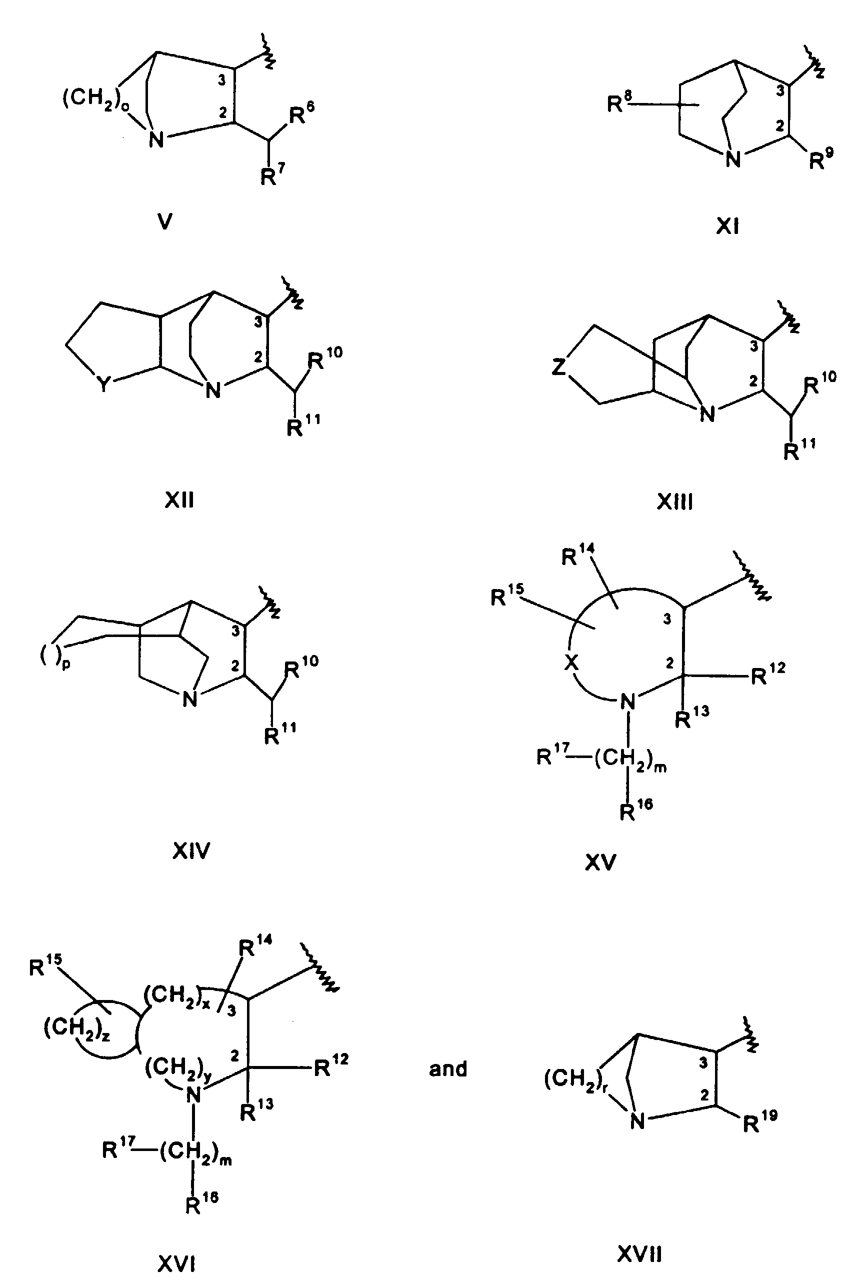
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁴ is (C₁-C₆) alkyl or phenyl;
R⁷ as selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein l is an integer from one to three, or Y is a group of the formula 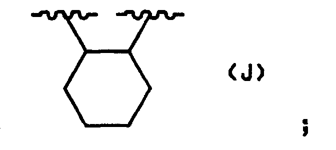
Z is oxygen, sulfur, amino, (C₁-C₃)alkylamino or (CH₂)ₙ, wherein n is zero, one or two;
x is zero, one or two;
y is zero, one or two;
z is three, four or five;
o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆) alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-, (C₁-C₆)alkyl-, di-(C₁-C₆)alkylamino, -C(=O)-NH-(C₁-C₆)alkyl, (C₁-C₆)-alkyl-C(=O)-NH-(C₁-C₆)alkyl, -NHC(=O)H and -NHC(=O)-(C₁-C₆)alkyl; and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to such point of attachment may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C(=O)-(C₁-C₆)alkyl-, and the radicals set forth in the definition of R¹²;
R¹⁶ is NHC(=O)R¹⁸, NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
with the proviso that (a) when m is 0, one of R¹⁶ and R¹⁷ is absent and the other is hydrogen, (b) when R³ is a group of the formula XVI, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ can not both be hydrogen, and (e) when R¹⁴ or R¹⁵ is attached to a carbon atom of X or (CH₂)_{y}, that is adjacent to the ring nitrogen, then R¹⁴ or R¹⁵, respectively, must be a substituent wherein the point of attachment is a carbon atom.

4. A pharmaceutical composition according to claim 1, wherein the NK-1 receptor antagonist or pharmaceutically acceptable salt thereof is selected from compounds of the formula XVIII, as depicted and defined below, and their pharmaceutically acceptable salts: 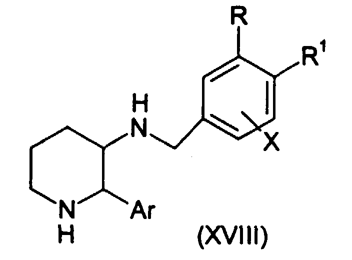
wherein R is halo (C₁-C₈)alkyl, halo (C₂-C₈)alkenyl, halo (C₂-C₈)alkynyl or halo (C₁-C₈)alkyl substituted by hydroxy or (C₁-C₈)alkoxy; R¹ is hydrogen, halo or (C₁-C₆)alkoxy; or
R and R¹, together with the two carbon atoms shared between the benzene ring and the R and R¹, complete a fused (C₄-C₆)cycloalkyl wherein one carbon atom is optionally replaced by oxygen and wherein one or two of the carbon atoms are optionally substituted by up to five subtituents selected from halo, (C₁-C₆)alkyl and halo (C₁-C₆)alkyl;
X is (C₁-C₆)alkoxy, halo (C₁-C₆)alkoxy, phenoxy or halo; and
Ar is phenyl optionally substituents by halo.

5. A pharmaceutical composition according to claim 1, wherein the NK-1 receptor antagonist or pharmaceutically acceptable salt thereof is selected from compounds of the formula XIX, as depicted and defined below, and their pharmaceutically acceptable salts: 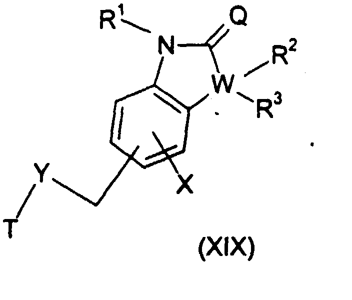 wherein
W is methylene, ethylene, propylene, vinylene, -CH₂-O-,-O-CH₂-, -CH₂-S- or -S-CH₂-;
R¹, R² and R³ are independently hydrogen, (C₁-C₃) alkyl, (C₁-C₃) alkoxy or halo (C₁-C₃) alkyl, provided that when W is methylene, both R² and R³ are not hydrogen;
X is halo, (C₁-C₃) alkoxy, (C₁-C₃) alkoxy or (C₁-C₃) alkenyl;
Y is imino or oxy;
Q is oxygen or sulfur; and
T is (2S,3S)-2-diphenylmethylquinuclidin-3-yl,(2S,3S)-2-phenylpiperdin-3-yl or (2S,3S)-2-diphenylmethyl-1-azanorbornan-3-yl.

6. A pharmaceutical composition according to claim 1, wherein the NK-1 receptor antagonist or pharmaceutically acceptable salt thereof is selected from compounds of the formula XX, as depicted and defined below, and their pharmaceutically acceptable salts: 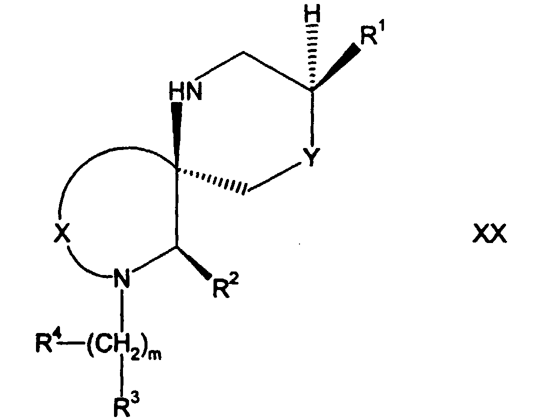
wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆)alkyl-C(=O)-NH-(C₁-C₆) alkyl, hydroxy(C₁-C₄)alkyl,-NHC(=O)H, -NHC(=O)-(C₁-C₆) alkyl, (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, (SO₂-(C₁-C₁₀)alkyl) ((C₁-C₁₀)alkyl)N wherein one or both of the alkyl moieties may optionally be substituted with from one to three fluorine atoms, -N(SO₂-(C₁-C₁₀)alkyl)₂ and (SO₃-aryl) ((C₁-C₁₀)alkyl)N; and wherein the aryl moieties of said -S(O)ᵥ-aryl, -O-aryl and (SO₂-aryl) ((C₁-C₁₀)alkyl)N are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 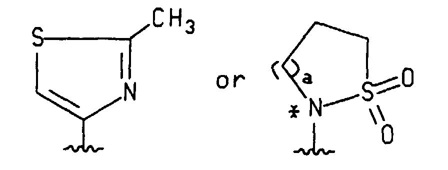
wherein a is 0, 1 or 2 and the asterisk represents a position meta to the point of attachment of R¹;
R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C ₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C-(C₁-C₆)alkyl-, di-(C₁-C₆)alkylamino, -C(=O)NH-(C₁-C₆)alkyl, (C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from NHC(=O)R⁸, NHCH₂R⁸, SO₂R⁸, AR⁵, CO₂H and the radicals set forth in the definitions of R², R⁶ and R⁷;
A is CH₂, nitrogen, oxygen, sulfur or carbonyl;
R⁸ is (C₁-C₆)alkyl, hydrogen, phenyl or phenyl (C₁-C₆)alkyl;
R⁴ is selected from oximino (=NOH) and the radicals set forth in the definitions of R², R⁶ and R⁷;
R⁵ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 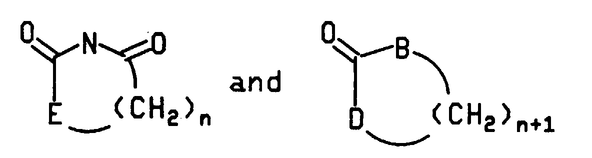
wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C-, (C₁-C₆)alkyl-C(=O)-(C₁-C₆)alkyl- and the radicals set forth in the definition of R²; and
Y is (CH₂)_{z} wherein z is zero or one;
with the proviso that (a) when A is -(CH₂)- or carbonyl, R⁵ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; (c) when R⁶ or R⁷ is attached to a carbon atom of X that is adjacent to the ring nitrogen, then R⁶ or R⁷, respectively, must be a substituent wherein the point of attachment is a carbon atom;

7. A pharmaceutical composition according to claim 1 wherein the amount of eletriptan or pharmaceutically acceptable salt thereof, in said composition is from about 0.1 to about 400 mg and the amount of the NK-1 receptor antagonist or pharmaceutically acceptable salt thereof, is from about 5 mg to about 200 mg.

8. The use of eletriptan, or a pharmaceutically acceptable salt or composition thereof, and a CNS-penetrant NK-1 receptor antagonist, or a pharmaceutically acceptable salt or composition thereof, for the manufacture of a medicament for simultaneous, separate or sequential use in the curative, palliative or prophylactic treatment of migraine.

9. Use according to claim 8, wherein the NK-1 receptor antagonist is a compound of the formula XX, as defined in claim 6, or pharmaceutically acceptable salt thereof.

10. Use according to claim 8, wherein the NK-1 receptor antagonist or pharmaceutically acceptable salt thereof is a compound of the formula I, as defined in claim 2, or pharmaceutically acceptable salt thereof.

11. Use according to claim 8, wherein eletriptan, or a pharmaceutically acceptable salt of eletriptan, and the NK-1 receptor antagonist or pharmaceutically acceptable salt thereof are administered as part of the same dosage form.

12. Use according to claim 8, wherein the NK-1 receptor antagonist is a compound of the formula XIa, as defined in claim 3, or pharmaceutically acceptable salt thereof.

13. Use according to claim 8, wherein the NK-1 receptor antagonist is a compound of the formula XIX, as defined in claim 5, or pharmaceutically acceptable salt thereof.

14. Use according to claim 8, wherein the NK-1 receptor antagonist is a compound of the formula XIb, as defined in claim 3, or pharmaceutically acceptable salt thereof.

15. Use according to claim 8, wherein the NK-1 receptor antagonist is a compound of the formula VIII, as defined in claim 4, or pharmaceutically acceptable salt thereof.

16. Use according to claim 8, wherein eletriptan, or pharmaceutically acceptable salt of eletriptan, and the NK-1 receptor antagonist are administered separately according to a dose regimen that renders the combination of the separately administered active agents effective in the treatment or prevention of migraine.

17. Use according to claim 8, wherein the NK-1 receptor antagonist is administered in an amount from about 5 to about 200 mg per day and eletriptan, or pharmaceutically acceptable salt thereof, is administered in an amount from about 0.1 to about 400 mg per day.

18. A pharmaceutical composition according to claim 2, wherein the NK-1 receptor antagonist or pharmaceutically acceptable salt thereof that is employed in such composition is selected from compounds of the formula I, as defined in the specification and in claim 2, and their pharmaceutically acceptable salts, with the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group.

19. Use according to claim 17, wherein the NK-1 receptor antagonist or pharmaceutically acceptable salt thereof that is employed in such use is selected from compounds of the formula I, as defined in the specification and in claim 17, and their pharmaceutically acceptable salts, with the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group.

20. A pharmaceutical composition comprising
(a) eletriptan, or a pharmaceutically acceptable salt thereof;
(b) a CNS-penetrant NK-1 receptor antagonist, or a pharmaceutically acceptable salt thereof; and, optionally,
(c) a pharmaceutically acceptable diluent, excipient or carrier.

21. A composition as claimed in claim 20 wherein the CNS-penetrant NK-1 receptor antagonist, or a pharmaceutically acceptable salt thereof, is as defined in any one of claims 2 to 6.

22. A pharmaceutical composition as claimed in claim 20 or 21 for use as a medicament.

23. The use of a pharmaceutical composition as claimed in claim 20 or 21 for the manufacture of a medicament for the curative, palliative or prophylactic treatment of migraine.

24. A product containing eletriptan, or a pharmaceutically acceptable salt or composition thereof, and a CNS-penetrant NK-1 receptor antagonist, or a pharmaceutically acceptable salt or composition thereof, as a combined preparation for simultaneous, separate or sequential use in the curative, palliative or prophylactic treatment of migraine.

25. A product as claimed in claim 24 wherein the CNS-penetrant NK-1 receptor antagonist, or a pharmaceutically acceptable salt thereof, is as defined in any one of claims 2 to 6.
